# EUROPEAN PATENT SPECIFICATION

(11) **EP 2 370 588 B1**
(45) Date of publication and mention of the grant of the patent: **30.04.2014**
(21) Application number: 09829397.0
(22) Date of filing: 27.11.2009
(51) Int. Cl.: C12P 7/64, C12N 5/04, A23L 1/03

(54) **Fatty acids and methods for producing them from Portulaca oleracea (purslane)**
Fettsäuren und Verfahren zu ihrer Herstellung aus Portulaca oleracea (Portulak)
Acides gras et procédés de fabrication de ceux-ci à partir de Portulaca oleracea (pourpier)

(30) Priority: 28.11.2008 SE 0802493
(43) Date of publication of application: 05.10.2011
(73) Proprietor: Brodelius, Maria, 393 53 Kalmar (SE); Olsson, Monica, 211 29 Malmö (SE)
(72) Inventor: Brodelius, Maria, 393 53 Kalmar (SE); Olsson, Monica, 211 29 Malmö (SE)
(74) Representative: Brann AB
(86) International application number: PCT/SE2009/000504
(87) International publication number: WO 2010/062236

(56) References cited:
- LORGERIL ET AL: "Alpha-linolenic acid and coronary heart disease", NUTRITION METABOLISM AND CARDIOVASCULAR DISEASES, vol. 14, 2004, pages 162-169, XP005118345,
- LIU ET AL: "Fatty acids and beta-carotene in Australian purslane (Portulaca oleracea) varieties", JOURNAL OF CHROMATOGRAPHY A, vol. 893, 2000, pages 207-213, XP004210389,
- TEIXEIRA ET AL: "Molecular cloning and expression analysis of three omega-6 desaturase genes from purslane (Portulaca oleracea L.)", BIOTECHNOLOGY LETTERS, vol. 31, 10 March 2009 (2009-03-10), pages 1089-1101, XP019670337,
- SAFDARI ET AL: "Plant tissue culture study on two different races of purslane (Portulaca oleracea L.)", AFRICAN JOURNAL OF BIOTECHNOLOGY, vol. 8, 2 November 2009 (2009-11-02), pages 5906-5912, XP002677320,
- BÖHM H. ET AL.: 'Establishment and characterization of a betaxanthin-producing cell culture from Portulaca grandiflora' PLANT CELL, TISSUE AND ORGAN CULTURE vol. 26, 1991, pages 75 - 82, XP003026499
- DATABASE WPI Week 199443, Derwent Publications Ltd., London, GB; XP003026500 & JP 06 087785 B (NISSHIN OIL MILLS LTD) 09 November 1994
- SIMOPOULOS A. P. ET AL: 'Common Purslane: A Source of Omega-3 Fatty Acids and Antioxidants' JOURNAL OF THE AMERICAN COLLEGE OF NUTRITION vol. 11, no. 4, 1992, pages 374 - 382, XP003026901
- ROSSI-HASSANI B.-D. ET AL: 'In vitro culture and plant regeneration of large flowered purslane' PLANT CELL, TISSUE AND ORGAN CULTURE vol. 41, 1995, pages 281 - 283, XP003026902
- YAMAMOTO K. ET AL: 'Isolation and Purification of Tyrosine Hydroxylase from Callus Cultures of Portulaca grandiflora' PLANT CELL PHYSIOLOGY vol. 42, no. 9, 2001, pages 969 - 975, XP003026903
- DATABASE WPI Week 198740, Derwent Publications Ltd., London, GB; XP003026904 & JP 62 195291 A (NITTO ELECTRIC IND CO) 28 August 1987
- FONTANA E. ET AL: 'Nitrogen concentration and nitrate/ammonium ratio affect yield and change the oxalic acid concentration and fatty acid profile of purslane (Portulaca oleracea L.) grown in a soilless culture system' JOURNAL OF THE SCIENCE OF FOOD AND AGRICULTURE vol. 86, 2006, pages 2417 - 2424, XP003026905
- BRODELIUS ET AL: "Omega-3 fatty acid production in plant cell cultures", American Society of Plant Biologists; poster # P46030, 2009, Retrieved from the Internet: URL:http://abstracts.aspb.org/pb2009/publi c/P46/P46030.html
- TEIXEIRA: "Growth conditions, omega-3 fatty acid concentrations and gene expression of fatty acid desaturase in Portulaca oleracea leaves", Universidade do Algarve / thesis, 2009, Algarve, Portugal Retrieved from the Internet: URL:https://sapientia.ualg.pt/bitstream/10 400.1/2119/1/doctoral%20thesis%202010.pdf

## Description

### FIELD OF INVENTION

The present invention relates to a method of producing specific plant components including PUFAs (polyunsaturated fatty acids) and/or antioxidants, wherein said products are obtained from *Portulaca oleracea* liquid culture(s), wherein said cultures comprises differentiated structures of said chlorophyline plant material and/or red leaves.

### BACKGROUND OF THE INVENTION

PUFAs (polyunsaturated fatty acids) have well documented positive health effects. The dominating source of these fatty acids is various kinds of marine oils. Large efforts have been made to enrich PUFAs from marine oils to obtain more concentrated products for use as food supplements or food ingredients. However, often PUFAs derived from marine oils have an unpleasant taste and generally contains heavy metals and other contaminants. Natural sources of PUFAs tend to have a highly heterogenous oil composition and require extensive purification to separate one or more desired PUFAs.

The PUFAs are important components of the plasma membrane of the cell, where they may be found in such forms as phospholipids. PUFAs are necessary for proper development, particularly in the developing infant brain, and for tissue formation and repair. PUFAs also serve as precursors to other molecules of importance in human beings and animals, including the prostacyclins, eicosanoids, leukotrienes and prostaglandins. Two enzymes are involved in linoleic and linolenic acid biosynthesis; linoleic acid (LA, 18:2 ^{Δ9,12}) is produced from oleic acid (18:1^{Δ9}) by the action of a Δ12-desaturate, and α-linolenic acid (ALA, 18:3^{Δ9,12,15}) is produced from linoleic acid (18:2 ^{Δ9,12}) by the action of a Δ15-desaturase. However, animals cannot desaturate beyond the Δ9 position and therefore cannot convert oleic acid into linoleic acid. Likewise, α-linolenic acid cannot be synthesized by mammals. Other eukaryotes, including fungi and plants, have enzymes which desaturate at positions Δ12 and Δ15. The major polyunsaturated fatty acids of animals therefore are either derived from diet and/or from desaturation and elongation of linoleic or α-linolenic acids.

Linoleic and α-linolenic acids are essential fatty acids (EFA), which means that, like vitamins, they must be obtained in the diet. The essential nature of these PUFA was established in the 1930s (1). Linoleic acid was found to be the parent or precursor for a series of PUFA, now known as the n-6 PUFA since linoleic acid itself is an n-6 PUFA; likewise, α-linolenic acid (ALA) is the parent FA for the n-3 PUFA. α-Linolenic acid is called ALA in order to distinguish it from γ-linolenic acid, an n-6 PUFA found in evening primrose oil and borage oil. All FA in the linoleic acid (n-6) PUFA family has their first double bond 6 carbons from the terminal methyl end of the molecule. Similarly, all FA in the ALA (n-3) PUFA family has their first double bond 3 carbons from the methyl end.

Today more or less all the PUFA, i.e., DHA and EPA, available on the market are derived from marine material but there are also PUFA's that marine animals cannot produce/synthesise which may be of importance, such as linoleic and linolenic fatty acids. However, single cell oils (SCO) are now widely accepted in the market place and there is a growing awareness of the health benefits of PUFAs, such as g-linolenic acid (GLA), arachidonic acid (ARA),docosahexaenoic acid (DHA) and eicosapentaenoic acid (EPA). ARA and DHA have also been used for fortification of infant formulae in many parts of the world. Fish oils are rich sources of DHA and EPA and a limited number of plant oilseeds are good sources of other PUFAs. Marine protists and dinoflagellates, such as species of *Thraustochytrium, Schizochytrium* and *Crypthecodinium* are the rich sources of DHA, whereas microalgae like *Phaeodactylum* and *Monodus* are good sources of EPA. Species of lower fungi *Mortierella* accumulate a high percentage of ARA in the lipid fraction.

Böhm *et al* disclose cell cultures of yellow flowering *Portulaca grandiflora,* wherein said cultures are undifferentiated and wherein said cultures contain betacyanins.

Brodelius and Teixeira, on a poster abstract at a 2009 conference, disclose the development of a cell culture line for the production of PUFAs. This document is an intermediate document, i.e., published after the priority date but prior to the international filing date; see http://abstracts.aspb.org/pb2009/public/p46/p46030.html

Teixeira published in May 2009 her thesis, which discloses the growth of *Portulaca oleracea* leaves in cell cultures for the production of PUFAs. This document is an intermediate document, i.e., published after the priority date but prior to the international filing date; see http://sapientia.ualg.pt/ bitstream/10400.1/2119/1/doctoral%20thesis%202010.pdf

However, there is therefore a need to develop new methods to be able to produce specific PUFAs by alternative ways, to be able to produce more pure specific PUFAs being substantially free from heavy metals as well as having a bad taste or even to find a way to produce specific PUFAs which cannot be produced today.

### SUMMARY OF THE INVENTION

The invention relates to a method to produce specific plant components including PUFAs as well as antioxidants being purified from plant cell liquid cultures, wherein said plant cell liquid culture is the semi-differentiated structures obtained from in vitro cultures of *Portulaca oleracea*. Thereby it is for the first time possible to produce plant specific PUFAs such as n-6 PUFA linoleic acid (LA) and n-3 PUFA α-linolenic acid (ALA).

By the invented method there will be no problems with the occurrence of bad taste or the presence of heavy metals, since the production is under controlled conditions, as well as new PUFAs will be obtained having new characteristics.

The invention relates to a method of producing specific plant components including PUFAs and/or antioxidants comprising the steps of: providing seeds from *Portulaca oleracea*; cultivating said seeds under conditions that initiate chlorophyline embryos and/or red leaves, developing differentiating structures of said chlorophyline embryos and/or red leaves and growing said structures in large scale culture, collecting said structures, and obtaining specific plant components including PUFAs and/or antioxidants from said structures. By managing to transfer the chlorophyline calli into differentiated calli or embryos, i.e., starting the development of an organ it has surprisingly been found that the differentiated material produces PUFA as well as antioxidants in amount suitable for large scale production, which have not been shown before.

Thereby it is for the first time possible to produce new PUFAs being derived from plants, including LA and ALA, without genetic manipulation.

Further disclosed are PUFAs being derived by said method as well as product
comprising said PUFAs wherein said PUFA comprises ALA and LA in amount of 1:1 to 1:4, such as 1:2 or 1:3. The modem Western diet gives a wrong balance of omega 3:6 fatty acids into the human body. The majority of the population have in their food intake too high amounts of omega 6. Omeg 3 and omega 6 PUFAs compete for the same enzymes for conversion into long chain PUFAs. This means that a high intake of omega 6 will lead to further conversion of compounds of the omega 6 group which are related to promotion of inflammatory, heart and vascular diseases, and in consequence to this, a lower percentage of the omega 3 beneficial compounds will be present in the body. Most natural products containing PUFAs have higher proportions of omega 6 in comparison to omega 3, this is common in plant seeds, for example. In storage tissues of children with overweight ratios of omega 3:6 were found to be ca 1:30 whereas a normal ratio is considered to be closer to 1:5. To prevent the bad effects of such an unbalanced omega 6 intake, it is important to find products where the ration omega 3:6 will provide a much higher intake of omega 3 and, in a stable form that will not oxidase fast. In this new formed plant structure in liquid culture a ratio of 1:1 is available, and when consumed directly the PUFA molecules are integrated in organelles where they keep a stable form.

By the invented method it is for the first time possible to isolate specific PUFAs from the lipid fraction of a plant-derived structure obtained by *in vitro* production, i.e., isolated PUFA rich mixture in linoleic as well as linolenic x fatty acids. PUFAs that animals as well as mammals cannot produce and which needs to be derived from the diet. The isolated PUFAs can then be used as an additive in different food products, such as baby food, wherein there are high demands on the quality. The isolated PUFAs may also be used in other food x product such as yogurt, soya products, and juices, as well as a diet supplement in the form of capsules. Alternatively the isolated PUFAs can be used in cosmetic products were the demands are also high on the quality.

### BRIEF DESCRIPTION OF THE DRAWINGS

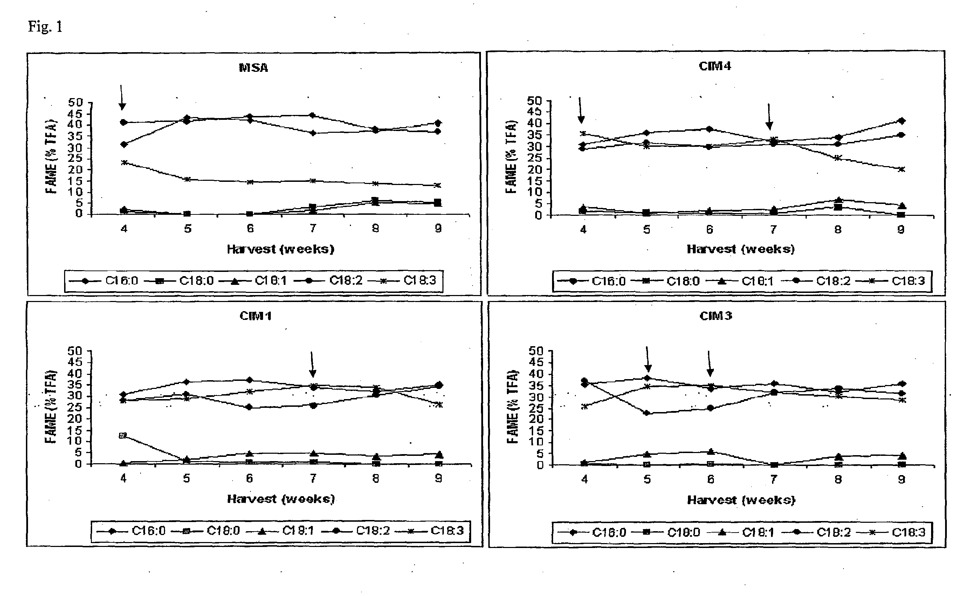

Fig. 1 Fatty acid accumulation in GCC cultures, in dry weight basis. The arrows indicate when the target fatty acids, LA and LNA, were detected in higher concentrations.
Fig. 2 show gas chromatograms of fatty acid methyl esters from embryo-like structures cultured in liquids MSA media. 1-myristic acid; 2-palmitic acid, 3-palmitoleic acid; 4-stearic acid; 5-oleic acid, 6-linoleic acid and 7-linolenic acid.

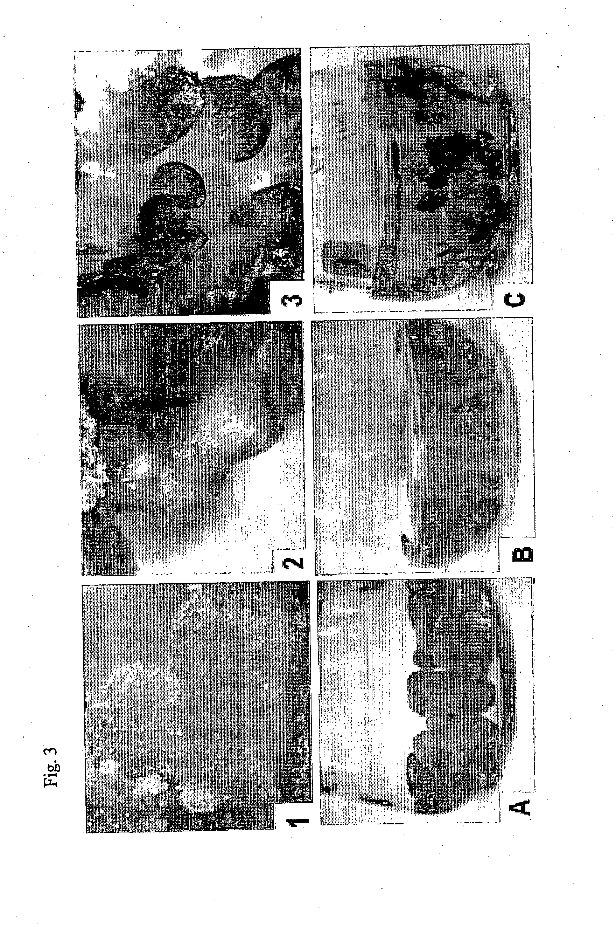

Fig 3 show calli development and structure differentiation from 4 week old leaf and stem explants of *P. oleracea* cultured in CIM, EMI and SFM solid media (1-3), and respective cell suspensions (A-C). Once transferred from solid media to liquid media, green calli (1) originated oval green structure (A) in CIM media, embryo-like structure (2) kept their structure and grown in embryo-like stuctures attached to the original embryo (B), in EIM; and red-leaf structures (3) differentiation was maintained in liquid media (C), in SFM.

### DETAILED DESCRIPTION OF THE INVENTION

### Definitions

The term "PUFA" is intended to mean polyunsaturated fatty acids, including the n-6 PUFA linoleic acid (LA) and n-3 PUFA α-linolenic acid (ALA), which only efficiently can be derived from specific plant species as mentioned below.

The term "green friable calli (GFC)" refers to somatic green calli (less than 0.1 mm of diameter) obtained from the plant explants (leaves or stems) in solid culture media. GFC being the same as green calli or chlorophyline calli.

The term "green compact embryo-like structure (GCES)" refers to a somatic embryo obtain from plant explants in solid culture media and/or cultured in liquid culture media. GCES being the same as chlorophylin embryos or green embryos.

The term "red leaf agglomerates (RLA)" or red leaves refers to a differentiated structure, composed by two or more red leaves connected together, obtained from the plant stem explants in solid culture media and/or cultured in liquid culture media.

The term "oval green structure (OGS)"refers to a semi-differentiated structure derived from green friable calli (GFC) cultured in liquid culture media. OGS being the same as pre-leaves chlorophyline.

### Method of the invention

Natural sources of PUFAs, such as animals and plants, tend to have highly heterogeneous oil compositions. The oils obtained from these sources therefore can require extensive purification to separate out one or more desired PUFAs or to produce oil which is enriched in one or more PUFA. Natural sources also are subject to uncontrollable fluctuations in availability. Fish stocks may undergo natural variation or may be depleted by over-fishing. Fish oils have unpleasant tastes and odours, which may be impossible to economically separate from the desired product, and can render such products unacceptable as food supplements. Cropland available for production of alternate oil-producing crops is subject to competition from the steady expansion of human populations and the associated increased need for food production on the remaining arable land. Crops which do produce PUFAs, such as borage, have not been adapted to commercial growth and may not perform well in monoculture. Growth of such crops is thus not economically competitive where more profitable and better established crops can be grown. Large scale fermentation of organisms such as Mortierella is also expensive. Microorganism, such as Porphyridium and Mortierella, are difficult to cultivate on a commercial scale. A need exists for oils containing higher relative proportions of and/or enriched in specific PUFAs. A need further exists for reliable economical methods of producing specific PUFAs in a controlled reliable environment. In particular, there is a need to express A 12 and A 15 desaturases and PUFAs in a plant cell culture system. A plant cell system can be used to produce linoleic and linolenic acids in a controllable, reliable and natural system, by altering growth conditions, without the need to use recombinant methods of production.

The invention relates to a method of producing specific plant components, including PUFAs and/or antioxidants comprising the steps of: providing seeds from *Portulaca oleracea.* The seeds may be derived from the golden leaves as well as from green leaves varieties. Said seed may be surface disinfected in varies ways to remove microorganisms, such as fungi and bacteria. The seeds will then be germinated and growth in vitro on a suitable medium such as the medium disclosed in the EXAMPLES. The seeds are grown into seedlings and explants from those seedlings are further cultivated under conditions that initiate calli (GFC), green compact embryo-like structures (GCES) and/or red-leafs agglomerates (RLA) production. The GFC are then grown in large scale production in liquid media and new semi-differentiated structures (OGS) are developed. To drive the growth and development of these structures, a mixture of auxin and cytokine hormones was used. The content of the auxin was from 0 to about 1.0 mg/l and the content of cytokine was from about 0.5 to about 1.0 mg/l. Examples of the auxin content may be 0, 0.05, 0.06, 0.07, 0.08, 0.09, 0.1, 0.2, 0.3, 0.4, 0.5, 0.6, 0.7 or 1.0 mg/l and examples of intervals may be 0.05-0.5, 0.1-1.0 or 0.1-0.6 mg/l. Examples of the cytokine content may be 0.5, 0.55, 0.6, 0.65, 0.7, 0.75, 0.8, 0.85, 0.9, 0.95, 1.0 mg/l and examples of intervals may be 0.6-1.0, 0.5-0.9 or 0.7-0.8 mg/l.

After growth of the CES and/or OGS, they will be harvested and the specific PUFAs obtained from these structures by any suitable means well known for a person skilled in the art such as what is mentioned under the EXAMPLES. Examples of specific plants PUFAs are n-6 PUFA linoleic acid (LA) and n-3 PUFA α-linolenic acid (ALA), which are present in high amount in the above mentioned species. The amount of LA is at least 3 mg/g fresh tissue, such as at least 3.5 mg/fresh tissue and the amount of ALA is at least 1 mg/fresh tissue such as 1.1 mg/fresh tissue (Tables 1). The relationship between the amounts of ALA and LA being about 1:1 to 1:4, such as 1:2 or 1:3.

Further disclosed are PUFAs being obtained from said method, wherein it is the first time they may be purified in an efficient and economical way, since these PUFAs cannot be produced in marine animals and cannot be derived from marine species without the presence of heavy metals. The method uses cell culture in liquid media from plant-derivated stuctures obtained from explants of *Portulaca oleracea* by *in vitro* production. Since the culture conditions are controlled and the nutrients are added in a known composition, according to the plant needs, in a sterile environment, no contamination with heavy metals or any other contaminants are present. Also no genetic manipulation is applied. These PUFAs are synthesized and accumulated naturally in the original plant tissues.The PUFAs may be ALA and/or LA or other green plant originating PUFAs

### Advantages of plant suspension cell culture versus plant crop:

Average linoleic acid content in purslane (*Portulaca oleracea*) leaves is about 1.87 mg LA/g FW (18% of total lipid), and 4.51mg/g FW linolenic acid (43%), witch is higher than the amounts produced in cell culture, but cell culture provides a controlled, reliable system that provides sufficient plant material in only 4 weeks time (opposing to the plant that requires 6 to 8 weeks to reach adult stage when higher levels of LA and ALA are obtained). Explants from one plant can originate a large number of the structures cultivated in cell suspension, witch also is able to grow and multiply under these conditions. Also, this plant is considered a semi-tropical plant that requires temperature of 25-30°C and long periods of light to grow, witch enables the establishment of this culture in more cold countries or it requires the use of glass greenhouses, witch would increase production costs. Suspension cell cultures are maintained in sterile conditions, under controlled and reproducible conditions of light, temperature and surrounding atmosphere, being able to be produced and maintained in any facility with an incubation chamber.

### Advantages of plant suspension cell culture versus recombinant expression in microorganisms:

No genetic manipulation is involved in this process, since these structures are obtained from the leaves and/or stems of a plant that naturally is rich in these fatty acids. So the use of this system would not raise problems in public opinion. Preliminary studies showed that this system could be economically competitive with the microbiological systems with genetic recombinant expression of these enzymes

### EXAMPLES

### EXAMPLE 1

### Production of the cell cultures

Seeds of *Portulaca oleracea* from the golden leaves and green leaves varieties were surface disinfected with 10% (w/v) sodium hypochlorite for 10 minutes, washed with sterile water and placed for germination and growth in vitro, on basal MS medium (Murashige & Skoog, 1962), solidified with 0.6% (w/v) Difco purified agar. All explants were taken from these in vitro cultures. Calli initiation and maintenance medium consisted of MS medium supplemented with 2% (w/v) sucrose and 0.6% (w/v) Difco purified agar (pH 5.8). All tested media were supplemented with vitamins (1 mg/l thiamine, 100 mg/ myo-inositol, 0.5 mg/l pyridoxine, 0.5 mg/l nicotinic acid) and growth regulators. Explants, 2-3 mm in length, were taken from 1-month-old *in vitro-grown* plants and were placed on solid MS medium supplemented with combinations of growth regulators. Cultures were maintained under long day conditions, 16-h-light, 27 °C day/21 °C night. Three main structures were produced from the explants (not shown). Leaf explants originated green friable callus (GFC) and green compact embryo-like callus (GCES), and stem explants originated red-leafs agglomerates (RLA). These structures were sub-cultured at 3-weeks intervals onto the same media. A number of GFC, GCES and RLA were transferred and maintained in CIM, EIM and SFM liquid media, respectively, and incubated in Kühner Shaker, 1SF-1-W model (Switzerland) regulated for 16h light period, at 26 °C, with smooth agitation. GFC cultures differentiated forming an oval greenish structure (OGS), in suspension cultures capable of growing and subdivide; GCES and RLA retain their original form and develop new similar structures attached to the original structure. If separated, these structures survive and continue to grow forming new structures

### EXAMPLE 2

### Analysis of the fatty acid profile of the suspension of cell cultures

To evaluate the feasibility of this application, the fatty acid composition of OGS and GCES was analysed. The OGS and the GCES Fatty acid profile were similar. The same fatty acids were present in both samples: palmitic (C16:0), stearic (C18:0), oleic (C18:1 ^{Δ9}), linoleic (C18:2^{Δ9,12}) and linolenic (C18:3 ^{Δ9, 12, 15}) were identified (Table 1). The OGS had a high content of linoleic acid (C18:2^{Δ9,12}), 3.5 mg LA/g fresh tissue (43% of the total lipid content) and a relatively high content of linolenic acid, 1.1 mg ALA/g fresh tissue (13% of total lipids). GCES had lower amounts of both fatty acid, around ⅓ of LA and ½ ALA, compared to the oval green structures, although the proportion ALA/LA was higher (Table 1). In further studies, we decided to test different hormonal combination in the liquid media, to access its effect in the structure of the OGS and fatty acid accumulation, more specifically linoleic and linolenic acids. GFC were cultured in 4 media with different auxin/ cytokine proportions: MSA (0.05 mg/l NAA: 0.5 mg/l BAP (1:10)), CIM4 (0.5 mg/l NAA: 1.0 mg/l BAP (1:2)), CIM1 (: 0.5 mg/l NAA: 0.5 mg/l BAP (1:1)), and CIM3 (1.0 mg/l NAA: 0.5 mg/l BAP (2:1)). The cultures were incubated in a Kühner Shaker, 1SF-1-W model (Switzerland) regulated for 16h light period, at 26 °C, with smooth agitation for 9 weeks. In all cultures, GFC cultures differentiated forming oval green structures (OGS) in liquid media culture, but with different sizes and colours.

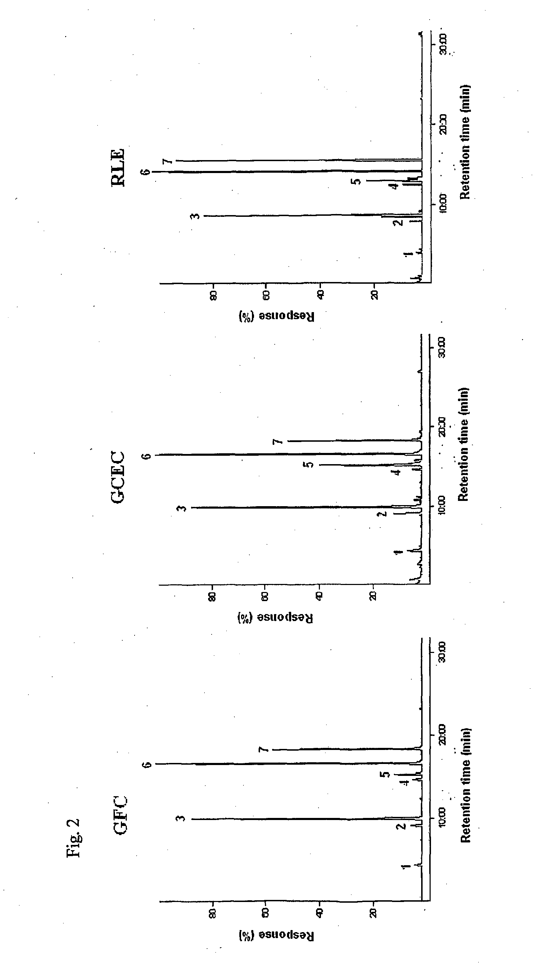

In the MSA medium, with the auxin/cytokine (NAA/BAP) ratio of 1:10, we obtained the largest structures 1-2 cm diameter, with a dark green colour. CIM4 (1:2 NAA/BAP) and CIM1 (1:1 NAA/BAP) produced structures with similar sizes, approximately 0.5 cm diameter, but different colour; CIM4 produced bright green structures and CIM1 produced light green structures. The medium that produced the smaller structures (0.1 cm diameter) was the CIM3, whereas the auxin/cytokine ratio was 2:1. This data suggest that the hormone proportion in medium affects the development of these structures, and that higher auxin values negatively affect the dimension of the formed structures. These structures slowly grown during the first 7 weeks, and started to show signs of stress at the ninth week, when the assay was ended. Samples were taken every week and fatty acid accumulation was determined in fresh and dry callus. Palmitic (C16:0), linoleic (C18:2^{Δ9,12}) and linolenic (C18:3^{Δ9,12,15}) constituted the major fraction of fatty acids present in the cell cultures (Table 2), (Figl), (Fig2). Stearic (C18:0) and oleic (C18:1^{Δ9}) acid were also detected, but at very low concentrations (Table 2). In almost all cultures, independently of hormones concentration, the highest amounts of target fatty acids were achieved between 6 and 7 weeks of culture (Table 2). Unfortunately all cultures had also high amounts of the saturated palmitoleic acid, in similar or even higher proportion that the target fatty acids, LA and LNA, that needs to be removed from the lipid mixture in further purification procedures in order to obtain an oil extract enriched in LA and ALA. In CIM4 the highest amount of LA and LNA was detected in the fourth week, and declined during the assay, which suggests that the harvest should have been done in the second or third week of culture. CIM3 and CIM4 mediums had almost equivalent amounts of LA and LNA, with the highest value at the fourth and sixth week of culture, respectively, with an ALA/LA ratio of one. LA accumulation was 2-fold higher than ALA in MSA cultures, and, in contrast, the CIM1 had higher accumulation of ALA (Table 2).

To summarize, if the objective is to produce oil with a high ALA/LA ratio, the CIM1 medium should be used. In the other hand, MSA cultures will provide oil with lower ALA/LA, but the produced biomass, in the same period of time will be greater. CIM3 and CIM4 produced equal amounts of LA and LNA, but in terms of biomass and culture time, the CIM4 medium offers better results.

**Table 1.Fatty acid composition of purslane cell suspension cultures. ¹ Fatty acids concentration, mgFA/g FW; ² Fatty acid relative proportion in sample (% in oil); ³ ALA/LA ratio**

| **Sample** | **Concentration (mg FA/g leaves, FW basis)¹** | | | | | | | **Relative percentage (%)²** | | | | | | **ALA/LA³** |
|---|---|---|---|---|---|---|---|---|---|---|---|---|---|---|
| | **C16:0** | **C16:1** | **C18:0** | **C18:1** | **C18:2** | **C18:3** | **Total** | **C16:0** | **C16:1** | **C18:0** | **C18:1** | **C18:2** | **C18:3** | |
| Oval green structures (OGS) | 1.12 | 2.55 | Residual | residual | 3.52 | 1.08 | 8.28 | 14 | 31 | residual | residual | 43 | 13 | 0.31 |
| Embryo-like structures (GCES) | 0.54 | 0.86 | Residual | residual | 1,11 | 0.62 | 3.13 | 17 | 28 | residual | residual | 36 | 20 | 0.56 |
| Portulaca oleracea leafs | 2.26 | 0.19 | 0.56 | 1.07 | 1.87 | 4.51 | 10.46 | 22 | 2 | 5 | 10 | 18 | 43 | 2.41 |

**Table 2. Fatty acid composition of purslane cell suspension cultures. A - Fatty acids concentration in dry weight basis (DW) mg FA/g culture (DW); ²Fatty acid relative proportion in sample (% in oil);³ Unsaturated/saturated ratio; ⁴ ALA/LA ratio. PA-palmitoleic acid C16:0; SA-stearic acid C18:0; OA-oleic acid C18:1^{Δ9}; LA-linoleic acid C18:2^{Δ9,12}; ALA - α-linolenic acid C18:3^{Δ9,12,15}.**

| **Sample** | **Harvest (week)** | **Concentration(mg FAME/g tissue DW** | | | | | | **Concentration (% in oil)** | | | | | **Insaturated/saturated³** | **ALA/LA⁴** |
|---|---|---|---|---|---|---|---|---|---|---|---|---|---|---|
| | | **PA** | **SA** | **OA** | **LA** | **ALA** | **Total** | **PA** | **SA** | **OA** | **LA** | **ALA** | | |
| MSA | 4 | 5,83 | 0,42 | 0,46 | 5,96 | 3,54 | 16,21 | 36,0 | 2,6 | 2,8 | 36,8 | 21,8 | 1,6 | 0,6 |
| | 5 | 5,48 | 0,58 | 0,54 | 6,21 | 3,35 | 16,16 | 33,9 | 3,6 | 3,3 | 38,4 | 20,7 | 1,7 | 0,5 |
| | 6 | 5,28 | 0,77 | 0,71 | 6,60 | 3,14 | 16,50 | 32,0 | 4,7 | 4,3 | 40,0 | 19,0 | 1,7 | 0,5 |
| | 7 | 6,61 | 0,60 | 0,68 | 7,36 | 3,34 | 18,59 | 35,6 | 3,2 | 3,7 | 39,6 | 18,0 | 1,6 | 0,5 |
| | 8 | 5,20 | 0,55 | 0,52 | 4,57 | 1,22 | 12,06 | 43,1 | 4,6 | 4,3 | 37,9 | 10,1 | 1,1 | 0,3 |
| | 9 | 4,89 | 0,27 | 0,65 | 2,92 | 1,70 | 10,43 | 46,9 | 2,6 | 6,2 | 28,0 | 16,3 | 1,0 | 0,6 |

## Claims

1. A method of producing specific plant components including PUFAs (polyunsaturated fally acids) and/or antioxidants comprising the steps of:
a) providing seeds from the *Portulaca oleracea,*
b) cultivating said seeds under conditions that initiate chlorophyline embryos and/or red leaves production,
c) developing differentiating structures of said chlorophyline embryos and/or red leaves and growing said structures in large scale culture,
d) collecting said structures, and
e) obtaining specific plant components including PUFAs and/or antioxidants from said structures.

2. The method according to any if claims 1, wherein said specific plants PUFAs comprises n-6 PUFA linoleic acid (LA) and n-3 PUFA α-linolenic acid (ALA).

3. The method according to claim 1-2, wherein said amount of ALA:LA is about 1:1 to about 1:4.

4. The method according to any of preceding claims, wherein the structures formation is controlled by the auxin/cytokine ratio and content.

5. The method according to any of preceding claims, wherein the content of the auxin is from about 0.05 to about 1.0 mg/l and the content of cytokine is from about 0.5 to about 1.0 mg/l.

6. The method according to any of preceding claims, wherein the LA content is at least 3 mg/g fresh tissue.

7. The method according to any of preceding claims, wherein the ALA content is at least 1 mg/g fresh tissue.

## Patentansprüche

1. Verfahren zur Herstellung spezifischer Pflanzenkomponenten, die mehrfach ungesättigte Fettsäuren (PUFAs) und/oder Antioxidantien enthalten, das die folgenden Schritte umfasst:
a) Bereitstellung von Samen der *Portulaca oleracea,*
b) Kultivierung der Samen unter Bedingungen, die die Herstellung von chlorophyllhaltigen Embryonen und/oder roten Blättern einleiten,
c) Entwicklung sich differenzierender Strukturen der chlorophyllhaltigen Embryonen und/oder der roten Blätter und Ziehen der Strukturen in Kultur in großem Maßstab,
d) Sammeln der Strukturen, und
e) Gewinnen spezifischer Pflanzenkomponenten, die PUFAs und/oder Antioxidantien enthalten, aus den Strukturen.

2. Verfahren gemäß Anspruch 1, worin die spezifischen Pflanzen-PUFAs n-6 PUFA Linolsäure (LA) und n-3 PUFA α-Linolensäure (ALA) umfassen.

3. Verfahren gemäß Anspruch 1 bis 2, worin die Menge von ALA:LA ungefähr 1:1 bis ungefähr 1:4 beträgt.

4. Verfahren gemäß irgendeinem vorhergehenden Anspruch, worin die Ausbildung der Strukturen durch das Auxin/Cytokin-Verhältnis und den Auxin/Cytokin-Gehalt kontrolliert ist.

5. Verfahren gemäß irgendeinem vorhergehenden Anspruch, worin sich der Auxin-Gehalt im Bereich von ungefähr 0.05 bis ungefähr 1.0 mg/L befindet und sich der Cytokin-Gehalt im Bereich von ungefähr 0.5 bis ungefähr 1.0 mg/L befindet.

6. Verfahren gemäß irgendeinem vorhergehenden Anspruch, worin der LA-Gehalt mindestens 3mg pro Gramm frischen Gewebes beträgt.

7. Verfahren gemäß irgendeinem vorhergehenden Anspruch, worin der ALA-Gehalt mindestens 1mg pro Gramm frischen Gewebes beträgt.

## Revendications

1. Procédé de production de composants végétaux spécifiques incluant des PUFA (acides gras polyinsaturés) et/ou des antioxydants comprenant les étapes consistant à :
a) fournir des graines issues de *Portulaca oleracea,*
b) cultiver lesdites graines dans des conditions qui initient la production d'embryons de chlorophyline et/ou de feuilles rouges,
c) développer des structures de différenciation desdits embryons de chlorophyline et/ou desdites feuilles rouges et faire pousser lesdites structures en culture à grande échelle,
d) collecter lesdites structures, et
e) obtenir des composants végétaux spécifiques incluant des PUFA et/ou des antioxydants à partir desdites structures.

2. Procédé selon la revendication 1, dans lequel lesdits PUFA végétaux spécifiques comprennent le PUFA n-6 acide linoléique (LA) et le PUFA n-3 acide α-linolénique (ALA).

3. Procédé selon les revendications 1 et 2, dans lequel ladite quantité d'ALA/LA est d'environ 1/1 à environ 1/4.

4. Procédé selon l'une quelconque des revendications précédentes, dans lequel la formation de structures est commandée par la teneur et le rapport auxine/cytokine.

5. Procédé selon l'une quelconque des revendications précédentes, dans lequel la teneur en auxine est d'environ 0,05 à environ 1,0 mg/L et la teneur en cytokine est d'environ 0,5 à environ 1,0 mg/L.

6. Procédé selon l'une quelconque des revendications précédentes, dans lequel la teneur en LA est d'au moins 3 mg/g de tissu frais.

7. Procédé selon l'une quelconque des revendications précédentes, dans lequel la teneur en ALA est d'au moins 1 mg/g de tissu frais.
